# EUROPEAN PATENT APPLICATION

(11) **EP 4 778 464 A1**
(43) Date of publication of application: **22.07.2026**
(21) Application number: 24894677.4
(22) Date of filing: 22.11.2024
(51) Int. Cl.: A61B 5/346, A61B 5/00

(54) **ELECTROCARDIOGRAM READING METHOD, PROGRAM AND DEVICE USING ARTIFICIAL INTELLIGENCE**

(30) Priority: 22.11.2023 KR 20230163830
(71) Applicant: Medical AI Co., Ltd., Seoul 06180 (KR)
(72) Inventor: KWON, Joonmyoung, Seoul 06180 (KR); LEE, Minsung, Seoul 06180 (KR)
(74) Representative: Patentanwaltskanzlei Matschnig & Forsthuber OG
(86) International application number: PCT/KR2024/018597
(87) International publication number: WO 2025/110786

(57) **Abstract**

According to an embodiment of the present disclosure, a method, a program, and a device for reading an electrocardiogram (ECG) using artificial intelligence, performed by a computing device, are provided. The method may comprise: acquiring ECG data from an ECG measurement device; performing a first classification according to readability of the acquired ECG data, based on analysis of metadata related to noise of the acquired ECG data; and performing a second classification, in response to a determination that the acquired ECG data is valid data requiring additional analysis according to the first classification, according to readability of the valid data, based on analysis of an ECG signal included in the valid data.

## Description

### TECHNICAL FIELD

The present disclosure relates to artificial intelligence (hereinafter, "AI") technology in the medical field, and more specifically, to a method for selecting a readable electrocardiogram (hereinafter, "ECG") by considering different characteristics of each ECG measurement device, and analyzing a disease or a health condition using the selected ECG.

### BACKGROUND OF THE INVENTION

An ECG is a vital biosignal that is important for diagnosing and monitoring a cardiac condition, and plays an essential role in modern medicine. Due to recent advancements in wearable devices, accessibility to ECG data has been significantly improved, enabling personal health monitoring not only in a medical setting but also in a daily life. In particular, a cloud-based ECG reading service has been established as a powerful tool that efficiently collects, processes, and analyzes ECG data to provide useful information to a user and a medical professional.

However, diversity in hardware characteristics and noise processing methods among ECG measurement devices causes several problems when such a service is provided to a user. Due to characteristics such as signal quality, noise removal methods, and data formats that differ for each device, it is difficult for conventional services to properly select and analyze a readable ECG regardless of device type and to provide consistent results. In particular, if characteristics of each device are not considered in noise determination and data selection processes, diagnostic accuracy and reliability may be degraded. Accordingly, because many conventional ECG analysis services are developed in a manner optimized for a specific device or do not analyze data by considering different data collection and processing characteristics for each device, uniform service quality cannot be guaranteed regardless of device type.

### SUMMARY OF THE INVENTION

### TECHNICAL PROBLEM

An object of the present disclosure is to provide an integrated system that enables consistent ECG data processing and analysis, regardless of characteristics and types of various ECG measurement devices.

However, the problems to be solved by the present disclosure are not limited to the aforementioned problems, and other unmentioned problems can be clearly understood from the following description.

### TECHNICAL SOLUTION

According to an embodiment of the present disclosure for achieving the aforementioned objects, a method for predicting a prognosis of heart failure, performed by a computing device, is provided. The method may comprise: acquiring ECG data from an ECG measurement device; performing a first classification according to readability of the acquired ECG data, based on analysis of metadata related to noise of the acquired ECG data; and performing a second classification, in response to a determination that the acquired ECG data is valid data requiring additional analysis according to the first classification, according to readability of the valid data, based on analysis of an ECG signal included in the valid data.

Alternatively, the metadata related to the noise of the ECG data may vary according to the type of the ECG measurement device.

Alternatively, the analysis of the metadata related to the noise of the acquired ECG data may comprise analyzing presence or absence of the metadata related to the noise, and an expression included in the metadata related to the noise.

Alternatively, the performing of the first classification according to the readability of the acquired ECG data, based on the analysis of the metadata related to the noise of the acquired ECG data, may comprise determining the acquired ECG data as invalid data with low readability, when the metadata related to the noise is present and the metadata related to the noise includes an expression indicating unreadability.

Alternatively, the performing of the first classification according to the readability of the acquired ECG data, based on the analysis of the metadata related to the noise of the acquired ECG data, may comprise determining the acquired ECG data as data to be read or the valid data, when the metadata related to the noise is present and the metadata related to the noise does not include an expression indicating unreadability.

Alternatively, the performing of the first classification according to the readability of the acquired ECG data, based on the analysis of the metadata related to the noise of the acquired ECG data, may comprise determining the acquired ECG data as the valid data, when the metadata related to the noise is absent or it is not possible to verify whether the metadata is present.

Alternatively, the performing of the second classification, in response to the determination that the acquired ECG data is the valid data requiring additional analysis according to the first classification, according to the readability of the valid data, based on the analysis of the ECG signal included in the valid data, may comprise performing the second classification according to the readability of the valid data, using a first neural network model pre-trained based on training data labeled by a human reader who determines readability based on an AI reading result.

Alternatively, the performing of the second classification, in response to the determination that the acquired ECG data is the valid data requiring additional analysis according to the first classification, according to the readability of the valid data, based on the analysis of the ECG signal included in the valid data, may comprise performing the second classification according to the readability of the valid data, using a second neural network model pre-trained based on training data labeled by comparing a readability determined by a human reader and a readability determined by an AI.

Alternatively, the performing of the second classification, in response to the determination that the acquired ECG data is the valid data requiring additional analysis according to the first classification, according to the readability of the valid data, based on the analysis of the ECG signal included in the valid data, may comprise performing the second classification according to the readability of the valid data, using a third neural network model pre-trained to detect ECG lead reversal based on a correlation between ECG leads.

Alternatively, the performing of the second classification, in response to the determination that the acquired ECG data is the valid data requiring additional analysis according to the first classification, according to the readability of the valid data, based on the analysis of the ECG signal included in the valid data, may comprise performing the second classification according to the readability of the valid data, using a fourth neural network model pre-trained to estimate whether an ECG waveform has voltage levels within a normal range by comparing the voltage levels within a characteristic interval of the ECG waveform.

Alternatively, the performing of the second classification, in response to the determination that the acquired ECG data is the valid data requiring additional analysis according to the first classification, according to the readability of the valid data, based on the analysis of the ECG signal included in the valid data, may comprise, when the second classification is performed using a plurality of neural network models, performing the second classification according to the readability of the valid data by combining outputs of the plurality of neural network models.

Alternatively, when the second classification is performed using the plurality of neural network models, performing the second classification according to the readability of the valid data by combining the outputs of the plurality of neural network models may comprise reclassifying the valid data as invalid data with low readability in response to an estimation made by at least one of the plurality of neural network models that the valid data has low readability.

Alternatively, the method may further comprise, in response to a determination that the valid data is data to be read according to the second classification, performing a reading on the data to be read, using a fifth neural network model pre-trained to perform a prediction regarding a disease or a health condition based on characteristics of an ECG waveform.

According to an embodiment of the present disclosure for achieving the aforementioned objects, a computer program stored on a computer-readable storage medium is provided. The computer program, when executed by one or more processors, causes operations to be performed for predicting a prognosis of heart failure. The operations may comprise: acquiring ECG data from an ECG measurement device; performing a first classification according to readability of the acquired ECG data, based on analysis of metadata related to noise of the acquired ECG data; and performing a second classification, in response to a determination that the acquired ECG data is valid data requiring additional analysis according to the first classification, according to readability of the valid data, based on analysis of an ECG signal included in the valid data.

According to an embodiment of the present disclosure for achieving the aforementioned objects, a computing device for predicting a prognosis of heart failure is provided. The device may comprise: a processor including at least one core; a memory including program codes executable by the processor; and a network unit for acquiring ECG data from an ECG measurement device. The processor may be configured to: perform a first classification according to readability of the acquired ECG data, based on analysis of metadata related to noise of the acquired ECG data; and perform a second classification, in response to a determination that the acquired ECG data is valid data requiring additional analysis according to the first classification, according to readability of the valid data, based on analysis of an ECG signal included in the valid data.

### ADVANTAGEOUS EFFECTS

According to the method of the present disclosure, the quality of ECG data may be improved by performing optimized noise removal and signal refinement processes that consider signal processing characteristics of various ECG measurement devices. This may contribute to deriving more accurate reading results, thereby enhancing user reliability.

Furthermore, by processing data collected from various devices in an integrated manner within a single system, a user may receive the same level of ECG analysis service without being constrained by devices used by subjects whose ECG is measured.

### BRIEF DESCRIPTION OF THE DRAWINGS

FIG. 1 is a block diagram of a computing device according to an embodiment of the present disclosure.
FIG. 2 is a block diagram illustrating a first classification process according to an embodiment of the present disclosure.
FIG. 3 is a block diagram illustrating a second classification process according to an embodiment of the present disclosure.
FIG. 4 is a flowchart illustrating a method for reading an ECG using AI according to an embodiment of the present disclosure.

### DETAILED DESCRIPTION OF THE EMBODIMENTS

Hereinafter, embodiments of the present disclosure will be described in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and is not to be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" is to be understood to mean any of the natural inclusive permutations. For example, if "X uses A or B" is used in the present disclosure, unless otherwise specified or clear from the context, it may be interpreted as any of the cases where X uses A, X uses B, or X uses both A and B.

The term "and/or" as used in the present disclosure is to be understood to refer to and include all possible combinations of one or more of the associated listed concepts.

The terms "comprise" and/or "comprising" as used in the present disclosure are to be understood to mean the presence of a specific feature and/or component. However, the terms "comprise" and/or "comprising" are to be understood as not precluding the presence or addition of one or more other features, other components, and/or combinations thereof.

In the present disclosure, unless otherwise specified or clear from the context to indicate a singular form, generally the singular is to be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data through a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented through software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within the scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have problem-solving ability by optimizing parameters that connect nodes or neurons through learning. A neural network "model" may include a single neural network or a set of neural networks in which a plurality of neural networks are combined.

The foregoing description of the terms is for the purpose of aiding the understanding of the present disclosure. Therefore, it is to be noted that if the foregoing terms are not explicitly stated as limiting the content of the present disclosure, they are not used in a sense that limits the technical spirit of the content of the present disclosure.

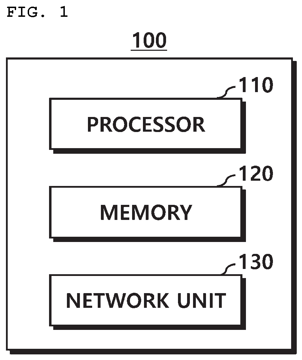

FIG. 1 is a block diagram of a computing device according to an embodiment of the present disclosure.

A computing device 100 according to an embodiment of the present disclosure may be a hardware device or part of a hardware device that performs comprehensive processing and calculation of data, or it may be a software-based computing environment connected via a communication network. For example, the computing device 100 may be a server, which is a subject that performs intensive data processing functions and shares resources, or it may be a client that shares resources through interaction with a server. In addition, the computing device 100 may be a cloud system in which a plurality of servers and clients interact to process data comprehensively. The above description is only one example related to the type of the computing device 100, and therefore, the type of the computing device 100 may be variously configured within the scope understandable by a person of ordinary skill in the art based on the content of the present disclosure.

Referring to FIG. 1, the computing device 100 according to an embodiment of the present disclosure may comprise a processor 110, a memory 120, and a network unit 130. However, FIG. 1 is only an example, and the computing device 100 may include other components to implement a computing environment. In addition, only some of the disclosed components may be included in the computing device 100.

The processor 110 according to an embodiment of the present disclosure may be understood as a component unit including hardware and/or software for performing computing operations. For example, the processor 110 may execute a computer program and perform data processing for machine learning. The processor 110 may process computational operations such as feature extraction for machine learning, error calculation based on backpropagation, and the like. The processor 110 for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor 110 described above are only examples, and the type of the processor 110 may be variously configured within the scope understandable by a person of ordinary skill in the art based on the content of the present disclosure.

The processor 110 may evaluate readability of ECG data through an analysis of metadata included in the ECG data collected from an ECG measurement device. Here, the readability may be understood as a probability indicating whether an ECG signal is a high-quality signal at a level that allows a determination of a health condition or a diagnosis of a disease through at least one of a human reader or AI. Information to be collected or a method for identifying and removing noise may differ for each type of ECG measurement device. Here, the type of the ECG measurement device may vary according to an ECG measurement method (e.g., single-lead measurement, 12-lead measurement, etc.) or may vary according to a manufacturer of the ECG measurement device. Accordingly, the processor 110 may analyze the metadata included in the ECG data to determine the readability of the ECG data by reflecting different data characteristics for each ECG measurement device. Through the analysis of the metadata included in the ECG data, the processor 110 may verify how signal quality was identified or how noise was processed in the device that measured the ECG data. Then, based on the verified information, the processor 110 may determine whether the ECG data is valid data requiring additional analysis or invalid data with low readability. Here, the low readability may be understood as a state in which the ECG signal itself is unreadable or a state in which a result of reading is predicted to have low accuracy or reliability even if the reading is performed.

In response to a determination that the ECG data is the valid data requiring additional analysis, the processor 110 may further evaluate the readability of the ECG data through an analysis of an ECG signal included in the ECG data. When primary filtering for the signal quality is completed through the metadata analysis considering characteristics for each type of the ECG measurement device, the processor 110 may perform secondary filtering for the signal quality by additionally performing an analysis on the ECG signal itself. For example, the processor 110 may estimate the readability of the valid data by evaluating a noise level of a signal included in the valid data as a continuous variable through a neural network model pre-trained to analyze the quality of an ECG signal. The processor 110 may estimate the readability of the valid data by analyzing whether a reversed lead signal is included in the valid data through a neural network model pre-trained to detect ECG lead reversal. The processor 110 may estimate the readability of the valid data by determining whether the voltage levels of a signal included in the valid data are within a normal range through a neural network model pre-trained to analyze voltage levels of an ECG signal. Here, the normal range of voltage levels may be a value pre-determined according to a clinical standard or a value set according to a user command. The processor 110 may perform at least one of the plurality of estimation methods for the readability described above. When performing two or more estimation methods, the processor 110 may derive a final result by combining estimation results for the readability of each method. Here, the combination may be implemented by an average operation on the estimation results, a weighted sum operation (e.g., a weighted sum in which a weight is assigned to each output according to importance set based on the frequency of use of each of a plurality of models), or the like. Through such a two-step filtering process, the processor 110 may accurately select readable ECG data and improve the quality of a reading service.

A memory 120 according to an embodiment of the present disclosure may be understood as a constituent unit including hardware and/or software for storing and managing data processed in a computing device 100. That is, the memory 120 may store any form of data generated or determined by the processor 110 and any form of data received by the network unit 130. For example, the memory 120 may include at least one storage medium of a flash memory type, a hard disk type, a multimedia card micro type, a card type memory, a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. In addition, the memory 120 may include a database system that controls and manages data in a predetermined system. The foregoing types of the memory 120 are only one example, and the type of the memory 120 may be variously configured within a scope understandable by a person of ordinary skill in the art based on the content of the present disclosure.

The memory 120 may structure, organize, and manage data necessary for the processor 110 to perform operations, a combination of data, and program codes executable by the processor 110. For example, the memory 120 may store medical data received through the network unit 130, which will be described later. The memory 120 may store program codes for operating a machine learning model to receive medical data and perform learning, program codes for operating the machine learning model to receive medical data and perform inference according to an intended use of the computing device 100, and processed data generated as the program codes are executed.

The network unit 130 according to an embodiment of the present disclosure may be understood as a constituent unit that transmits and receives data through any form of known wired/wireless communication system. For example, the network unit 130 may perform data transmission and reception using a wired/wireless communication system such as a local area network (LAN), wideband code division multiple access (WCDMA), long term evolution (LTE), wireless broadband internet (WiBro), 5th generation mobile communication (5G), ultra wide-band, ZigBee, radio frequency (RF) communication, wireless LAN, wireless fidelity (Wi-Fi), near field communication (NFC), or Bluetooth. Since the communication systems described above are only examples, the wired/wireless communication system for data transmission and reception of the network unit 130 may be variously applied in addition to the examples described above.

The network unit 130 may receive data necessary for the processor 110 to perform operations through wired or wireless communication with an arbitrary system, an arbitrary client, or the like. In addition, the network unit 130 may transmit data generated through the operations of the processor 110 through wired or wireless communication with an arbitrary system, an arbitrary client, or the like. For example, the network unit 130 may receive medical data through communication with a database within a medical environment, a cloud server that performs tasks such as standardization of medical data, a client such as a smart watch, or a medical computing device. The network unit 130 may transmit output data of a machine learning model, intermediate data derived during the operations of the processor 110, processed data, and the like through communication with the aforementioned database, server, client, or computing device.

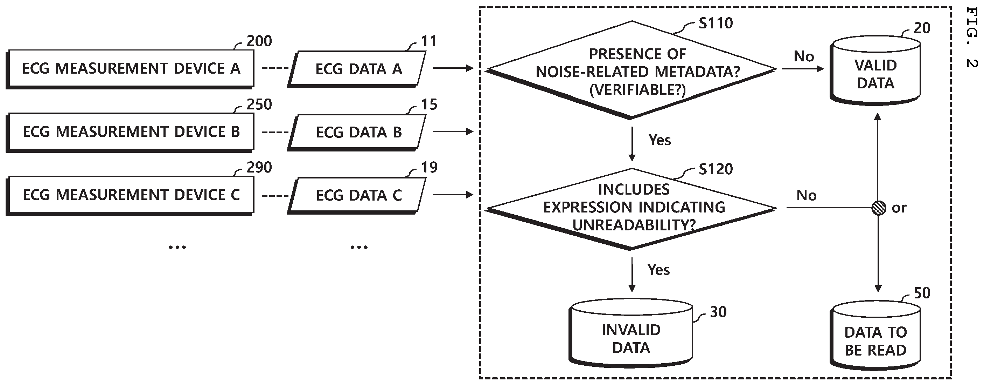

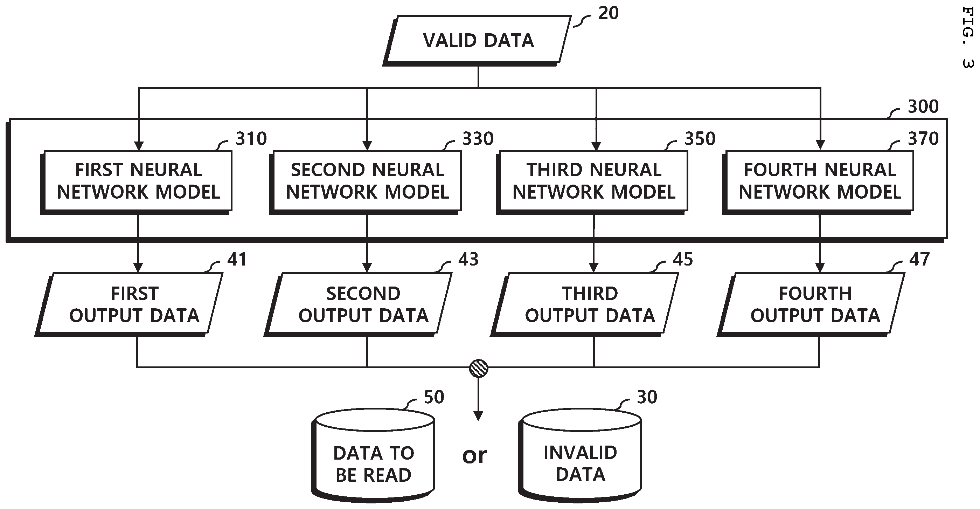

FIG. 2 is a block diagram illustrating a first classification process according to an embodiment of the present disclosure. FIG. 3 is a block diagram illustrating a second classification process according to an embodiment of the present disclosure.

The computing device 100 according to an embodiment of the present disclosure may acquire ECG data from an ECG measurement device. The computing device 100 may acquire the ECG data, which is collected and processed in the ECG measurement device, through communication with the ECG measurement device regardless of the type of the ECG measurement device. For example, referring to FIG. 2, the computing device 100 may perform communication with an ECG measurement device A 200, an ECG measurement device B 250, and an ECG measurement device C 290, respectively, to acquire ECG data A 11, ECG data B 15, and ECG data C 19. Here, the ECG measurement device A 200, the ECG measurement device B 250, and the ECG measurement device C 290 may be distinguished according to manufacturers. In addition, the ECG data A 11, the ECG data B 15, and the ECG data C 19 may be data in an XML format including an ECG signal and metadata.

The computing device 100 may perform the first classification according to the readability of the ECG data, based on analysis of metadata related to noise of the ECG data acquired from the ECG measurement device. Among the metadata included in the ECG data, the computing device 100 may analyze the metadata related to the noise to classify the ECG data into the valid data requiring additional analysis, the invalid data with low readability, or data to be read according to the readability of the ECG data. Here, the metadata related to the noise may be understood as metadata including information regarding noise of an ECG signal generated by the ECG measurement device.

Specifically, the computing device 100 may analyze presence or absence of the metadata related to the noise (S110). Each ECG measurement device may identify noise on its own or may not identify noise. In addition, even if the metadata related to the noise exists for each ECG measurement device, a case may occur where information included in the metadata cannot be verified due to various reasons. Accordingly, the computing device 100 may determine whether the noise-related metadata generated by the ECG measurement device is present and is in a state where verification is possible. When the noise-related metadata is absent in the ECG data or is in a state where verification is impossible, evaluation of quality through an analysis of the ECG signal must be performed, and thus the computing device 100 may first classify the ECG data as the valid data 20 requiring additional analysis. If the noise-related metadata is present in the ECG data in a state where verification is possible, the computing device 100 may perform an analysis on an expression included in the noise-related metadata.

The computing device 100 may analyze a noise-related expression included in the noise-related metadata. Specifically, the computing device 100 may analyze whether the noise-related metadata includes an expression indicating unreadability (S120). Here, the expression indicating unreadability may be understood as an expression including a concept of a state in which a result of reading a disease or a health condition is highly likely to have low accuracy or reliability. When the ECG measurement device itself generates noise information through an analysis of noise, an expression indicating whether signal quality is at a readable level may be included in the metadata. By identifying the corresponding expression, the computing device 100 may verify whether the ECG measurement device itself has determined the data as unreadable, thereby evaluating the readability of the ECG data. When the noise-related metadata includes the expression indicating unreadability, the computing device 100 may classify the ECG data as the invalid data 30 with low readability. That is, when the ECG measurement device determines the data as unreadable data, the computing device 100 may also classify the corresponding data as the invalid data 30 and decide not to use it for reading. When the noise-related metadata does not include the expression indicating unreadability, the computing device 100 may classify the ECG data as the valid data 20 requiring additional analysis or the data to be read 50. Since the ECG measurement device has determined that the ECG data is in a readable state, it may be immediately classified as the data to be read 50 without additional analysis. However, for a more accurate reading, additional analysis may be required even if the ECG measurement device has determined that the data is in a readable state. Accordingly, when the metadata related to the noise is present in the ECG data and the metadata related to the noise does not include the expression indicating unreadability, the computing device 100 may selectively classify the ECG data as the valid data 20 or the data to be read 50 according to a user command. For example, when metadata included in the ECG data in the XML format includes metadata information related to expressions such as "poor recording", "noise", "missing", "inconclusive", and the like, the computing device 100 may identify the aforementioned expressions and metadata information associated therewith to classify the corresponding data as the invalid data 30. When the metadata included in the ECG data in the XML format does not include the metadata information related to the aforementioned expressions, the computing device 100 may basically classify the corresponding data as the data to be read 50. Here, if pre-set to classify the data as the valid data 20 by the user command, the computing device 100 may classify the corresponding data as the valid data 20.

Through such first classification, the computing device 100 may primarily select readable data without the need to evaluate the quality of signals included in all ECG data collected from the ECG measurement device. That is, the computing device 100 may efficiently optimize computing resources required for data selection for reading through the first classification.

In response to a determination that the ECG data acquired from the ECG measurement device is the valid data 20 requiring additional analysis according to the first classification, the computing device 100 may perform the second classification according to the readability of the valid data 20, based on analysis of an ECG signal included in the valid data 20. The analysis of the ECG signal included in the valid data 20 may be understood as an operation of analyzing signal quality based on features existing in the ECG signal or a correlation between signals for each lead, using a pre-trained neural network model. For example, referring to FIG. 3, the computing device 100 may input the valid data 20 into a pre-trained neural network model 300 to generate output data regarding the readability of the valid data 20. Then, based on the output data of the neural network model 300, the computing device 100 may reclassify the valid data 20 as the invalid data 30 with low readability or classify it as the data to be read 50 to be used for reading.

Specifically, the computing device 100 may generate first output data 41 regarding the readability of the valid data 20 by inputting the valid data 20 into a first neural network model 310 pre-trained based on training data labeled by a human reader who determines readability based on an AI reading result. When there are ECGs determined to be readable and ECGs determined to be unreadable when determined by a human reader with reference to the AI reading result, labeling for the ECGs may be performed according to each determination to prepare training data for the first neural network model 310. The first neural network model 310 may be trained based on the training data prepared in this manner. When the training of the first neural network model 310 is completed, the computing device 100 may generate a probability value indicating the readability of the valid data 20 as the first output data 41 by inputting the valid data 20 into the trained first neural network model 310.

The computing device 100 may generate second output data 43 regarding the readability of the valid data 20 by inputting the valid data 20 into a second neural network model 330 pre-trained based on training data labeled by comparing a readability determined by a human reader and a readability determined by an AI. While the first neural network model 310 is trained based on training data labeled by determining whether reading is possible from a human's perspective, the second neural network model 330 differs in that it is trained based on training data labeled by determining whether reading is possible from an AI's perspective. For example, for data determined as unreadable due to noise from a human reader's perspective, if an AI reading is possible, the corresponding data may be labeled as readable data and included in the training data of the second neural network model 330. That is, the second neural network model 330 may be trained through data labeled by having a different perspective on determining readability from the first neural network model 310. Accordingly, the first neural network model 310 and the second neural network model 330 may be used complementarily. When the training of the second neural network model 330 is completed, the computing device 100 may generate a probability value indicating the readability of the valid data 20 as the second output data 43 by inputting the valid data 20 into the trained second neural network model 330.

The computing device 100 may generate third output data 45 regarding the readability of the valid data 20 by inputting the valid data 20 into a third neural network model 350 pre-trained to detect ECG lead reversal based on a correlation between ECG leads. The ECG lead reversal occurs when sensors are placed in wrong positions during ECG measurement, and may cause problems such as measurement of an unreadable signal or an increased possibility of a false reading even if a reading is possible. That is, since the ECG lead reversal causes an error in ECG reading, it is preferable to classify a signal in which lead reversal has occurred as data with low readability. For this processing, the third neural network model 350 may be trained to derive a correlation existing between ECG leads and to output a probability that reversal occurs in a specific lead based on the derived correlation. Here, training may be performed in a supervised learning manner using training data in which lead reversal is labeled. When the training of the third neural network model 350 is completed, the computing device 100 may generate a probability value that a signal in which lead reversal has occurred exists in the valid data 20 as the third output data 45 by inputting the valid data 20 into the trained third neural network model 330.

The computing device 100 may generate fourth output data 47 regarding the readability of the valid data 20 by inputting the valid data 20 into a fourth neural network model 370 pre-trained to estimate whether an ECG waveform has voltage levels within a normal range by comparing the voltage levels within a characteristic interval of the ECG waveform. When the voltage levels of the ECG signal are small, it is difficult for an analysis of the ECG to be performed accurately. Accordingly, in order to allow measures such as amplifying the ECG signal with the small voltage levels or inducing measurement in another way to be taken, it is necessary to classify a signal having voltage levels smaller than the normal range as data with low readability. For this processing, the fourth neural network model 370 may be trained to detect a characteristic interval such as QRS in the ECG waveform, calculate a difference between the highest voltage point and the lowest voltage point of within the detected characteristic interval, and determine whether the calculated difference is included in the normal range. Here, the normal range may be voltage levels determined as clinically readable or a value set by a user request. In addition, training of the fourth neural network model 370 may be performed through supervised learning as well as unsupervised learning, self-supervised learning, and the like. When the training of the fourth neural network model 370 is completed, the computing device 100 may generate the fourth output data 47 including information on whether a signal included in the valid data 20 is a low-voltage signal out of the normal range by inputting the valid data 20 into the trained fourth neural network model 370.

As shown in FIG. 3, when a plurality of output data 41, 43, 45, and 47 are generated through a plurality of neural network models 310, 330, 350, and 370, the computing device 100 may perform the second classification on the valid data by combining result values included in the plurality of output data 41, 43, 45, and 47. When any of the plurality of output data 41, 43, 45, and 47 includes a result value corresponding to a determination that the readability of the valid data is low, the computing device 100 may reclassify the valid data 20 as the invalid data 30 with low readability. For example, while a probability value indicating the readability for the valid data 20 included in the first output data 41 is less than a pre-set reference value, when result values included in other output data 43, 45, and 47 correspond to a determination that the readability is high, the computing device 100 may reclassify the valid data 20 as the invalid data 30 with low readability. Conversely, when all of the plurality of output data 41, 43, 45, and 47 include result values corresponding to a determination that the readability of the valid data is high, the computing device 100 may classify the valid data 20 as the data to be read 50.

Meanwhile, as shown in FIG. 3, the second classification may be performed using all of the plurality of neural network models 310, 330, 350, and 370, but the second classification may also be performed using some of the plurality of neural network models 310, 330, 350, and 370. That is, the readability for the valid data 20 may be determined using only one of the plurality of neural network models 310, 330, 350, and 370 according to the user request, or the readability may be determined using all or some of them. Such selective operation has an advantage that data selection according to the user request is possible. In addition, since it is not necessary to use all models unconditionally to select the data to be read 50, such selective operation may contribute to efficient use of computing resources.

Through such second classification, the computing device 100 may accurately select high-quality data to be used for reading, thereby increasing the quality and reliability of a reading service. In addition, the computing device 100 may process data collected from various devices with a single integrated system regardless of types of devices, thereby providing an efficient reading service environment to the user.

In response to a determination that the valid data 20 is the data to be read 50 according to the second classification, the computing device 100 may perform reading on the data to be read 50 using a fifth neural network model pre-trained to perform a prediction regarding a disease or a health condition based on characteristics of an ECG waveform. For example, the computing device 100 may generate a probability value regarding the possibility of existence of structural heart disease as a reading result by inputting the data to be read 50 into the fifth neural network model pre-trained to analyze the possibility of existence of structural heart disease based on morphological features of the ECG waveform. Here, the fifth neural network model may be a model trained based on training data composed of ECG signals labeled with the presence or absence of structural heart disease. Meanwhile, a reading task of the fifth neural network model is not limited to structural heart disease, which is the aforementioned example, and may be expanded to various diagnostic areas that can be read by ECG.

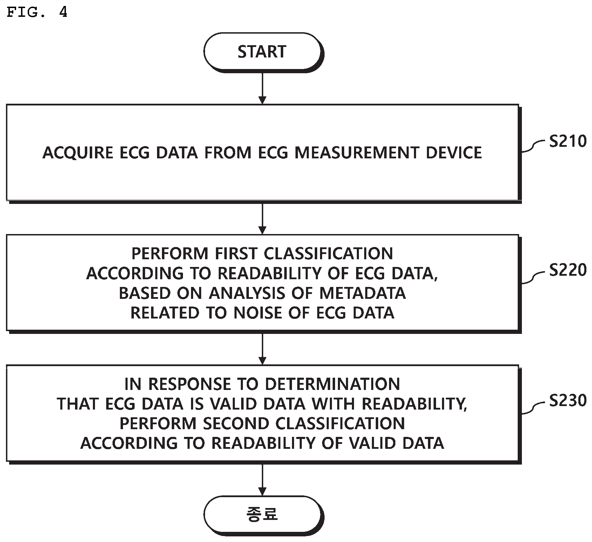

FIG. 4 is a flowchart illustrating a method for reading an ECG using AI according to an embodiment of the present disclosure.

Referring to FIG. 4, the computing device 100 according to an embodiment of the present disclosure may acquire ECG data from an ECG measurement device (S210). For example, the computing device 100 may receive the ECG data generated in the ECG measurement device through communication with the ECG measurement device. The ECG data generated in the ECG measurement device may include not only an ECG signal but also various metadata generated by the ECG measurement device itself.

The computing device 100 may perform the first classification according to readability of the acquired ECG data based on analysis of metadata related to noise of the ECG data acquired through step S210. Here, the analysis of the metadata related to the noise of the ECG data may be understood as analyzing presence or absence of the metadata related to the noise and an expression included in the metadata related to the noise. For example, when the metadata related to the noise is present and the metadata related to the noise includes an expression indicating unreadability, the computing device 100 may determine the ECG data as the invalid data with low readability. When the metadata related to the noise is present and the metadata related to the noise does not include an expression indicating unreadability, the computing device 100 may determine the ECG data as the data to be read or the valid data requiring additional analysis. When the metadata related to the noise is absent or it is impossible to verify whether the metadata is present, the computing device 100 may determine the ECG data as the valid data requiring additional analysis. Since the metadata related to the noise of the ECG data may vary according to the type of the ECG measurement device, the computing device 100 may quickly classify data by determining the readability through an analysis of the metadata as in the aforementioned example, even without directly evaluating the quality of the ECG signal.

In response to a determination that the ECG data acquired through step S210 is the valid data requiring additional analysis according to the first classification, the computing device 100 may perform the second classification according to the readability of the valid data based on analysis of an ECG signal included in the valid data (S230). The computing device 100 may perform the second classification according to the readability of the valid data, using the first neural network model pre-trained based on training data labeled by a human reader who determines readability based on an AI reading result. The computing device 100 may perform the second classification according to the readability of the valid data, using the second neural network model pre-trained based on training data labeled by comparing a readability determined by a human reader and a readability determined by an AI. The computing device 100 may perform the second classification according to the readability of the valid data, using the third neural network model pre-trained to detect the ECG lead reversal based on a correlation between ECG leads. The computing device 100 may perform the second classification according to the readability of the valid data, using the fourth neural network model pre-trained to estimate whether the ECG waveform has voltage levels within a normal range by comparing the voltage levels within a characteristic interval of the ECG waveform. The computing device 100 may perform the second classification by combining outputs generated using all or some of the four models described above, or may perform the second classification based on an output generated using any of the four models described above. For example, if any of the plurality of neural network models estimates that the valid data has low readability, the computing device 100 may reclassify the valid data as the invalid data with low readability. If all of the plurality of neural network models estimate that the valid data requires additional analysis, the computing device 100 may classify the valid data as the data to be read.

The various embodiments of the present disclosure described above may be combined with additional embodiments, and may be modified within the scope understandable by a person of ordinary skill in the art in light of the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts are to be interpreted as being included in the scope of the present disclosure.

## Claims

1. A method for reading an electrocardiogram (ECG) using artificial intelligence (AI), performed by a computing device including at least one processor, the method comprising:
acquiring ECG data from an ECG measurement device;
performing a first classification according to readability of the acquired ECG data, based on analysis of metadata related to noise of the acquired ECG data; and
performing a second classification, in response to a determination that the acquired ECG data is valid data requiring additional analysis according to the first classification, according to readability of the valid data, based on analysis of an ECG signal included in the valid data.

2. The method of claim 1, wherein the metadata related to the noise of the ECG data varies according to the type of the ECG measurement device.

3. The method of claim 1, wherein the analysis of the metadata related to the noise of the acquired ECG data comprises analyzing presence or absence of the metadata related to the noise, and an expression included in the metadata related to the noise.

4. The method of claim 3, wherein the performing of the first classification according to the readability of the acquired ECG data, based on the analysis of the metadata related to the noise of the acquired ECG data comprises:
determining the acquired ECG data as invalid data with low readability, when the metadata related to the noise is present and the metadata related to the noise includes an expression indicating unreadability.

5. The method of claim 3, wherein the performing of the first classification according to the readability of the acquired ECG data, based on the analysis of the metadata related to the noise of the acquired ECG data comprises:
determining the acquired ECG data as data to be read or the valid data, when the metadata related to the noise is present and the metadata related to the noise does not include an expression indicating unreadability.

6. The method of claim 3, wherein the performing of the first classification according to the readability of the acquired ECG data, based on the analysis of the metadata related to the noise of the acquired ECG data comprises:
determining the acquired ECG data as the valid data, when the metadata related to the noise is absent or it is not possible to verify whether the metadata is present.

7. The method of claim 1, wherein the performing of the second classification, in response to the determination that the acquired ECG data is the valid data requiring additional analysis according to the first classification, according to the readability of the valid data, based on the analysis of the ECG signal included in the valid data, comprises:
performing the second classification according to the readability of the valid data, using a first neural network model pre-trained based on training data labeled by a human reader who determines readability based on an AI reading result.

8. The method of claim 1, wherein the performing of the second classification, in response to the determination that the acquired ECG data is the valid data requiring additional analysis according to the first classification, according to the readability of the valid data, based on the analysis of the ECG signal included in the valid data, comprises:
performing the second classification according to the readability of the valid data, using a second neural network model pre-trained based on training data labeled by comparing a readability determined by a human reader and a readability determined by an AI.

9. The method of claim 1, wherein the performing of the second classification, in response to the determination that the acquired ECG data is the valid data requiring additional analysis according to the first classification, according to the readability of the valid data, based on the analysis of the ECG signal included in the valid data, comprises:
performing the second classification according to the readability of the valid data, using a third neural network model pre-trained to detect ECG lead reversal based on a correlation between ECG leads.

10. The method of claim 1, wherein the performing of the second classification, in response to the determination that the acquired ECG data is the valid data requiring additional analysis according to the first classification, according to the readability of the valid data, based on the analysis of the ECG signal included in the valid data, comprises:
performing the second classification according to the readability of the valid data, using a fourth neural network model pre-trained to estimate whether an ECG waveform has voltage levels within a normal range by comparing the voltage levels within a characteristic interval of the ECG waveform.

11. The method of claim 1, wherein the performing of the second classification, in response to the determination that the acquired ECG data is the valid data requiring additional analysis according to the first classification, according to the readability of the valid data, based on the analysis of the ECG signal included in the valid data, comprises:
when the second classification is performed using a plurality of neural network models, performing the second classification according to the readability of the valid data by combining outputs of the plurality of neural network models.

12. The method of claim 11, wherein, when the second classification is performed using the plurality of neural network models, performing the second classification according to the readability of the valid data by combining the outputs of the plurality of neural network models comprises:
reclassifying the valid data as invalid data with low readability in response to an estimation made by at least one of the plurality of neural network models that the valid data has low readability.

13. The method of claim 1, further comprising:
in response to a determination that the valid data is data to be read according to the second classification, performing a reading on the data to be read, using a fifth neural network model pre-trained to perform a prediction regarding a disease or a health condition based on characteristics of an ECG waveform.

14. A computer program stored on a computer-readable storage medium, the computer program, when executed by one or more processors, causing operations to be performed for reading an electrocardiogram (ECG) using artificial intelligence, the operations comprising:
acquiring ECG data from an ECG measurement device;
performing a first classification according to readability of the acquired ECG data, based on analysis of metadata related to noise of the acquired ECG data; and
performing a second classification, in response to a determination that the acquired ECG data is valid data requiring additional analysis according to the first classification, according to readability of the valid data, based on analysis of an ECG signal included in the valid data.

15. A computing device for reading an electrocardiogram (ECG) using artificial intelligence, the device comprising:
a processor including at least one core;
a memory including program codes executable by the processor; and
a network unit for acquiring ECG data from an ECG measurement device,
wherein the processor is configured to:
perform a first classification according to readability of the acquired ECG data, based on analysis of metadata related to noise of the acquired ECG data; and
perform a second classification, in response to a determination that the acquired ECG data is valid data requiring additional analysis according to the first classification, according to readability of the valid data, based on analysis of an ECG signal included in the valid data.
